# EUROPÄISCHE PATENTSCHRIFT

(11) **EP 1 991 865 B1**
(45) Veröffentlichungstag und Bekanntmachung des Hinweises auf die Patenterteilung: **22.05.2013**
(21) Anmeldenummer: 07704707.4
(22) Anmeldetag: 23.02.2007
(51) Int. Cl.: G01N 33/20, B21D 22/22, B21D 22/26

(54) **VORRICHTUNG ZUR PRÜFUNG DER QUALITÄT EINER METALLISCHEN BESCHICHTUNG**
DEVICE FOR TESTING THE QUALITY OF A METALLIC COATING
DISPOSITIF DE CONTRÔLE DE LA QUALITÉ D'UN REVÊTEMENT MÉTALLIQUE

(30) Priorität: 03.03.2006 DE 102006010431
(43) Veröffentlichungstag der Anmeldung: 19.11.2008
(73) Patentinhaber: ThyssenKrupp Steel Europe AG, 47166 Duisburg (DE)
(72) Erfinder: BIRKENSTOCK, Andreas, 42549 Velbert (DE); HEIDBÜCHEL, Peter, 47665 Sonsbeck (DE); LINNEPE, Michael, 59199 Bönen (DE)
(74) Vertreter: Cohausz & Florack
(86) Internationale Anmeldenummer: PCT/EP2007/051779
(87) Internationale Veröffentlichungsnummer: WO 2007/101795

(56) Entgegenhaltungen:
- EP-A1- 0 754 508
- US-A- 6 033 499
- M. GOLLE ET AL.: "Der Einfluss des Vorformens auf schwach konturierte Bauteile"[Online] 2001, XP002431278 Gefunden im Internet: URL:http://www.lfu.mb.uni-dortmund.de/page s/de/content/projekte/foerderung/spp1098/0 7_der_einfluss_des_vorformens_auf_schwach_ konturierte_bauteile_2001.pdf> [gefunden am 2007-04-26]
- "Blechumformung "under control"" ELEKTRO AUTOMATION, [Online] Bd. 54, Nr. 8, August 2001 (2001-08), Seiten 66-67, XP002431279 Gefunden im Internet: URL:http://www.logic-instrument.de/news/cl ippings/download/08_2001_ea.pdf> [gefunden am 2007-04-26]
- M. MERKLEIN ET AL.: "Entwicklung einer neuen Analysemethode für die Charakterisierung des Formgebungsvermögens metallischer Werkstoffe"[Online] 2003, Seiten 1-5, XP002431280 Gefunden im Internet: URL:http://www.utfscience.de/pdf/27202_UT- 3-01_003xx0103ut.pdf> [gefunden am 2007-04-26]

## Beschreibung

Die Erfindung betrifft eine Vorrichtung zur Bestimmung der Qualität einer metallischen Oberfläche eines metallischen Substrats mit einer Matrize, einem Blechhalter und einem Stempel, mit welcher das Substrat umgeformt wird, um eine gezogene Prüffläche herzustellen, wobei der Stempel so ausgebildet ist, dass im Bereich der Prüffläche des umgeformten Substrats die Hauptformänderung maximal 7 % und die Nebenformänderung zwischen -2 % und +2 %, vorzugsweise nahe 0 % beträgt.

Bei der Herstellung von Kraftfahrzeugen werden häufig Bleche verwendet, welche zur Erzielung bestimmter Eigenschaften, beispielsweise einer möglichst guten Korrosionsbeständigkeit, beschichtet sind. Die Beschichtung erfolgt in der Regel vor dem Umformprozess auf dem Substrat band- oder blechweise, so dass nicht nur das Metall selbst gute Umformeigenschaften aufweisen muss, sondern auch dessen Beschichtung. Ein typisches Beispiel für eine solche Beschichtung ist das Feuerverzinken von Stahlteilen, die beispielsweise für die Außenhaut eines Pkws eingesetzt wird. Die Beschichtungsqualität ist dabei von verschiedenen Parametern abhängig, so dass es wünschenswert ist, bereits im Vorfeld, d.h. vor der Weiterverarbeitung des Substrats beispielsweise zu Karosserieteilen, eine Überprüfung der Oberflächenqualität durchzuführen. Eine ähnliche Problematik betrifft aber auch unbeschichtete Bleche, beispielsweise Feinbleche, da beispielsweise Walzfehler häufig erst beim Umformen des Bleches zu einem Produkt auftreten. In diesem Fall ist es ebenfalls wünschenswert, eine die weitere Umformung des Bleches berücksichtigende Überprüfung der Oberflächenqualität durchzuführen.

Aus dem Stand der Technik sind eine Vielzahl von Verfahren bekannt, um die Oberflächenbeschaffenheit eines metallischen Substrats zu prüfen. Beispielsweise ist aus der DE 101 11 296 A1 bekannt, die Oberflächengüte eines Flachproduktes durch Auswertung von elektronischen Bildern der Oberfläche des Flachproduktes vorzunehmen. Informationen darüber, ob und für welche Umformprozesse die Oberfläche und/oder die Beschichtung geeignet ist, können mit dem bekannten Verfahren nicht gewonnen werden.

Aus dem gattungsgemäßen Aufsatz "Der Einfluss des Vorformens auf schwach konturierte Bauteile", M. Golle et al., XP-002431278 ist ein Verfahren und eine Vorrichtung zur Überprüfung der Formänderung und Formabweichung beim hydromechanischen Umformen bekannt.

Hiervon ausgehend liegt der vorliegenden Erfindung die Aufgabe zugrunde, eine gattungsgemäße Vorrichtung zur Bestimmung der Qualität einer metallischen Beschichtung auf einem metallischen Substrat zur Verfügung zu stellen, mit mit welcher die Qualität der metallischen Beschichtung anwendungsspezifisch, insbesondere im Hinblick auf eine spätere Umformung überprüft werden kann.

Gemäß der vorliegenden Erfindung wird die oben aufgezeigte Aufgabe durch eine gattungsgemäße Vorrichtung dadurch gelöst, dass der Stempel so ausgebildet ist, dass der Stempel eine Länge von mindestens 400 mm, eine Breite von mindestens 250 mm und eine Stirnfläche mit einer Krümmung in Richtung der Hauptformänderung mit einem Krümmungsradius von 500 bis 2000 mm, vorzugsweise 1000 mm aufweist.

Es hat sich herausgestellt, dass beispielsweise die in verschiedenen Bereichen eines Fahrzeuges eingesetzten Blechteile während der Herstellung unterschiedlich starken Umformungen unterzogen werden. Beispielsweise werden metallische Substrate für Dachkonstruktionen von Kraftfahrzeugen relativ geringen Hauptformänderungen von etwa 1 - 2 % ausgesetzt. Bei der Herstellung eines Blechteils für die Seitenwand eines Kraftfahrzeuges treten dagegen typischerweise Hauptformänderungen von ca. 5 % auf. Bei der Herstellung von Teilen für die Motorhaube eines Kraftfahrzeuges sind es beispielsweise in der Regel etwa 3 % Hauptformänderung. Mit der erfindungsgemäßen Vorrichtung werden nun die während der Weiterverarbeitung auftretenden Haupt- und Nebenformänderungen kontrolliert nachgebildet und anschließend die entsprechenden Haupt- und Nebenformänderungen aufweisende Prüffläche hinsichtlich der Qualität der metallischen Oberfläche überprüft. Die Überprüfung der metallischen Oberfläche der Prüffläche wird optisch, insbesondere unter Verwendung eines Mikroskops oder anderer optischer und/oder opto-elektronischer Vorrichtungen durchgeführt. Im Gegensatz zu dem aus dem Stand der Technik bekannten Vorrichtungen wird anwendungsspezifisch die Qualität der Oberfläche bestimmt, d. h. beispielsweise, ob das metallische Substrat einer bestimmten Umformung unterzogen werden kann, ohne dass es zu Fehlern in der metallischen Oberfläche kommt. Damit ist es prinzipiell möglich, die metallischen Substrate verschiedenen Umformungsanforderungen zuzuordnen und den Ausschussanteil bei der Herstellung von Konstruktionen für den Fahrzeugbau zu minimieren. Erfindungsgemäß weist der Stempel eine Länge von mindestens 400 mm und eine Breite von mindestens 250 mm auf, um eine möglichst große Prüffläche herzustellen. Die Prüffläche ist an die anwendungsspezifischen Größen der metallischen Substrate bei deren Verwendung im Fahrzeugbau angepasst, so dass die Bestimmung der Qualität der metallischen Beschichtung der Prüffläche eine repräsentative Aussage über die Qualität der Beschichtung des gesamten metallischen Substrats ermöglicht.

Erfindungsgemäß wird die Richtung der Hauptformänderung der Vorrichtung dadurch genau vorgegeben, dass der Stempel eine Stirnfläche mit einer Krümmung in Richtung der Hauptformänderung mit einem Krümmungsradius von 500 bis 2000 mm, vorzugsweise 1000 mm aufweist. Durch die Krümmung der Stirnfläche des Stempels wird beim Umformen, insbesondere beim Ziehen, die Hauptformänderung genau festgelegt. Aufgrund des großen Krümmungsradius erfolgt die Hauptformänderung sehr homogen über die gesamte Prüffläche.

Beträgt der Kantenradius zwischen der Stirnfläche des Stempels und zwei quer zur Hauptformänderungsrichtung verlaufenden Seitenflächen des Stempels 20 bis 80 mm, vorzugsweise 40 mm, wird ein Fließen des Materials des Substrats in Richtung der Hauptformänderung zusätzlich unterstützt. Die Prüffläche weist daher auch an ihren entsprechenden Randbereichen die über die Ziehtiefe eingestellte Hauptformänderung auf.

Die Nebenformänderung wird dagegen dadurch begrenzt, dass der Kantenradius zwischen der Stirnfläche des Stempels und zwei parallel zur Hauptformänderungsrichtung verlaufenden Seitenflächen des Stempels zwischen 2 und 10 mm, vorzugsweise 5 mm beträgt. Der scharfe Kantenradius verhindert im Wesentlichen eine Fließbewegung in Richtung der Nebenformänderung, d.h. quer zur Hauptformänderungsrichtung. Die Hauptformänderung sowie die Nebenformänderung wird damit auf einfache Weise fest vorgegeben.

Um den Einfluss der rechteckigen Form des Stempels auf die Prüffläche zu verringern weisen die Kanten zwischen den Seitenflächen des Stempels ein Kantenradius von 50 bis 100 mm, vorzugsweise 70 mm auf.

Die Bereiche des metallischen Substrats, die während des Ziehens an der Umformung teilnehmen, wird vorzugsweise dadurch begrenzt, dass die Matrize zwei senkrecht zur Richtung der Hauptformänderung verlaufende und beidseitig der Ausnehmung der Matrize angeordnete Sicken aufweist, die auch Ziehleisten genannt werden. Die Sicken unterdrücken, dass das Substrat außerhalb der Sicken zur Formänderung beitragen kann und ermöglicht damit, dass die Hauptformänderung über die gesamte Prüffläche nahezu konstant ist.

Schließlich ist es vorteilhaft, wenn verschiedene Ziehtiefen fest einstellbar sind. Über die fest einstellbaren Ziehtiefen können anwendungsspezifische Umformungen kontrolliert und reproduzierbar in das metallische Substrat eingebracht werden, so dass beispielsweise für die verschiedenen Anwendungsbereiche im Fahrzeugbau jeweils eine Ziehtiefe fest vorgegebenen werden kann.

Es gibt nun eine Vielzahl von Möglichkeiten die erfindungsgemäße Vorrichtung weiterzubilden und auszugestalten. Hierzu wird einerseits verwiesen auf die dem Patentanspruch 1 nachgeordneten Patentansprüche und andererseits auf die Beschreibung eines Ausführungsbeispiels einer erfindungsgemäßen Vorrichtung in Verbindung mit der Zeichnung. Die Zeichnung zeigt in
- Fig. 1a): eine Draufsicht auf einen Stempel eines Ausführungsbeispiels der erfindungsgemäßen Vorrichtung,
- Fig. 1b): den Stempel des Ausführungsbeispiels aus Fig. 1 in einer perspektivischen Darstellung,
- Fig. 2: eine Matrize des Ausführungsbeispiels der erfindungsgemäßen Vorrichtung aus Fig. 1 sowie
- Fig. 3: einen Blechhalter des Ausführungsbeispiels der erfindungsgemäßen Vorrichtung aus Fig. 1.

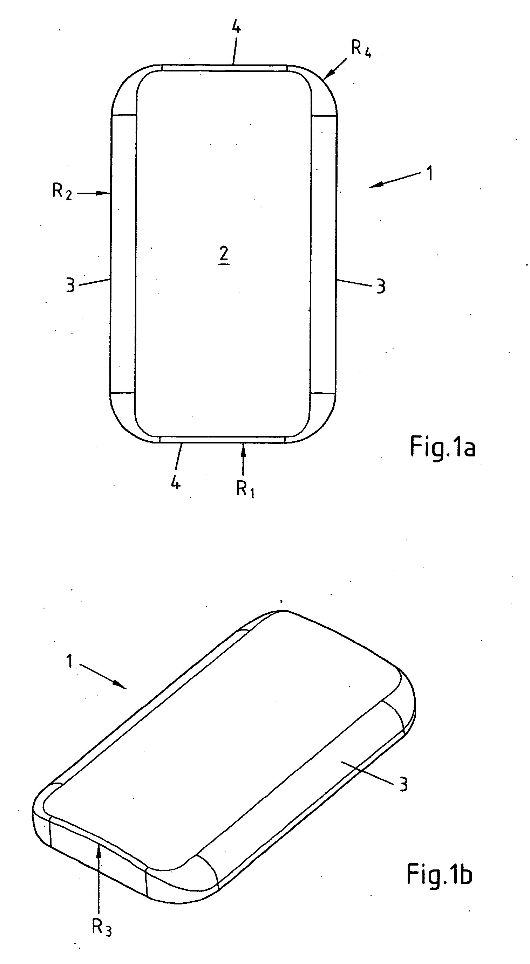

Fig. 1a) zeigt in einer Draufsicht den Stempel 1 eines Ausführungsbeispiels einer erfindungsgemäßen Vorrichtung zur Bestimmung der Qualität einer metallischen Beschichtung eines metallischen Substrats. Der Stempel 1 ist dabei so ausgebildet, dass die Stirnfläche 2 des Stempels beim Ziehen eines metallischen Substrats eine Prüffläche mit einer Hauptformänderung von maximal 7 % erzeugt. Einerseits weisen die Seitenkanten 3, 4 des Stempels 1 unterschiedliche Krümmungsradien auf, um das Fließverhalten des Materials im Wesentlichen in Richtung der Hauptformänderung zu beeinflussen. Andererseits betragen die Radien der Seitenkanten 4 im vorliegenden Ausführungsbeispiel 5 mm, so dass das Material des Substrats im Wesentlichen daran gehindert wird, über den Kantenradius hinaus zu fließen und eine Nebenformänderung zu erzeugen. Der Kantenradius der Seitenkanten 3 des Stempels 1 ist dagegen wesentlich größer, vorliegend beispielsweise 40 mm, um ein Fließen des Materials des Substrats über die Seitenkanten 3 hinaus zu ermöglichen. Hierdurch wird insbesondere in Verbindung mit dem Krümmungsradius R₃ der Stirnfläche 2 die Hauptformänderungsrichtung der Prüffläche vorbestimmt. beim Ziehen erfolgt somit eine Streckung des Materials im gesamten Bereich der Prüffläche von weniger als 7 %.

Die Länge und Breite des Stempels beträgt vorliegend 540 bzw. 320 mm, um repräsentative Ergebnisse im Hinblick auf die Überprüfung der Oberfläche der metallischen Beschichtung zu erzielen. Der Eckenradius R₄ zwischen den Seitenflächen des Stempels beträgt vorzugsweise 70 mm, um die aus den Ecken des Stempels 1 resultierenden Spannungen im Bereich der Prüffläche zu minimieren.

In der perspektivischen Darstellung des Stempels 1 in der Fig. 1b) wird deutlich, dass der Krümmungsradius des Stempels 1 R₃, welcher vorzugsweise zwischen 500 und 2000 mm, vorliegend 1000 mm beträgt, in einen relativ großen Kantenradius zur Seitenfläche 3 des Stempels 1 übergeht. Dieser Übergang beeinflusst das Streckverhaltens des metallischen Substrats positiv, so dass von den Seitenkanten 3 des Stempels 1 auf die Prüffläche 2 selbst kaum Einflüsse bzw. Änderungen der Hauptformänderung resultieren. Im Ergebnis weist die Prüffläche 2, welche der Stirnfläche des Stempels 1 entspricht, nahezu über den gesamten Bereich eine konstante Hauptformänderung in eine Richtung auf.

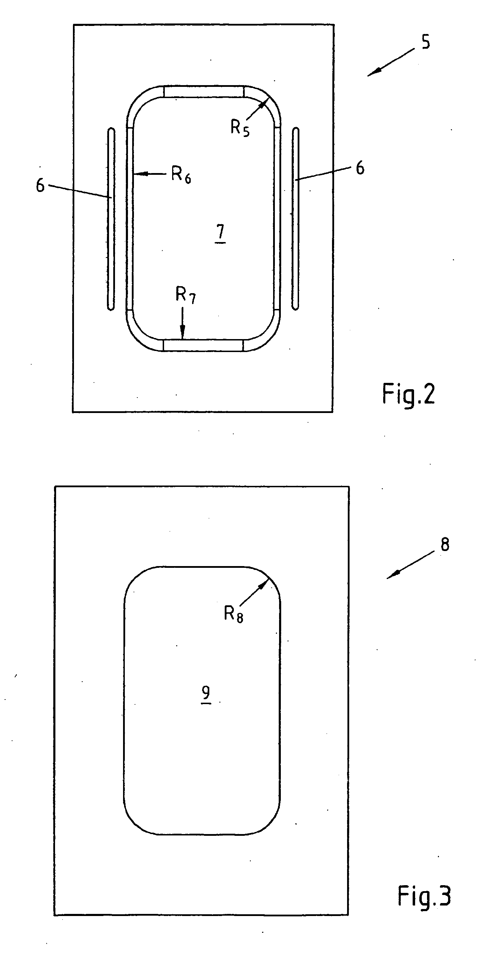

Fig. 2 zeigt nun in einer Draufsicht die zum Stempel 1 zugehörige Matrize 5. Deutlich zu erkennen sind die beiden parallel zur Ausnehmung der Matrize verlaufenden Sicken 6, welche verhindern sollen, dass zuviel Material aus weiter entfernten Bereichen beim Ziehen in den Ziehbereich hineinfließt. Die Sicken 6 verbessern die Homogenität der Hauptformänderung auf der Prüffläche, in dem diese das Nachfließen von Material aus weiter entfernten Bereichen des metallischen Substrates steuern. Die Matrize 5 weist analog zu dem Eckenradius R₄ des Stempels einen Eckenradius R₅ von ebenfalls vorzugsweise 70 mm auf. Zur definierten Einstellung der Hauptformänderung im Bereich der Prüffläche weist die Matrize 5 einen Kantenradius R₆ an der Längsseite der Ausnehmung 7 von beispielsweise 15 mm auf. Dieser Kantenradius unterstützt einerseits die Wirkung der Sicken, ermöglicht jedoch auch ein Fließen des Materials des metallischen Substrates, insbesondere bei Dicken des metallischen Substrats von 0,3 bis 1,5 mm. Der Kantenradius R₇ der Querkante der Ausnehmung kann größer gewählt werden, beispielsweise 25 mm, da an der Querkante der Ausnehmung im Wesentlichen ein Reißen des metallischen Substrats verhindert werden soll.

Fig. 3 zeigt nun den Blechhalter 8, welcher eine Ausnehmung 9 mit zur Stempelgeometrie passenden Abmessungen aufweist. Auch der Eckenradius R₈ der Ausnehmung 9 ist an die Geometrie des Stempels 1 angepasst und weist beispielsweise ebenfalls 70 mm auf.

## Patentansprüche

1. Vorrichtung zur Bestimmung der Qualität einer metallischen Oberfläche eines metallischen Substrats mit einer Matrize (5),
einem Blechhalter und einem Stempel (1), mit welcher das Substrat umgeformt wird, um eine gezogene Prüffläche herzustellen, wobei der Stempel (1) so ausgebildet ist, dass im Bereich der Prüffläche des umgeformten Substrats die Hauptformänderung maximal 7 % und die Nebenformänderung zwischen -2 % und +2 %, vorzugsweise nahe 0 % beträgt,
**dadurch gekennzeichnet, dass** der Stempel (1) eine Länge von mindestens 400 mm, eine Breite von mindestens 250 mm und eine Stirnfläche (2) mit einer Krümmung in Richtung der Hauptformänderung mit einem Krümmungsradius (R₃) von 500 bis 2000 mm, vorzugsweise 1000 mm aufweist.

2. Vorrichtung nach Anspruch 1,
**dadurch gekennzeichnet, dass** der Kantenradius (R₂) zwischen der Stirnfläche (2) des Stempels (1) und zwei quer zur Hauptformänderungsrichtung verlaufenden Seitenflächen (3) des Stempels 20 bis 80 mm, vorzugsweise 40 mm beträgt.

3. Vorrichtung nach einem der Ansprüche 1 oder 2,
**dadurch gekennzeichnet, dass** der Kantenradius (R₁) zwischen der Stirnfläche (2) des Stempels (1) und zwei parallel zur
Hauptformänderungsrichtung verlaufenden Seitenflächen (4) des Stempels zwischen 2 und 10 mm, vorzugsweise 5 mm beträgt.

4. Vorrichtung nach einem der Ansprüche 1 bis 3,
**dadurch gekennzeichnet, dass** die Kanten zwischen den Seitenflächen (3,4) des Stempels (1) einen Eckenradius (R₄) von 50 bis 100 mm, vorzugsweise 70 mm aufweisen.

5. Vorrichtung nach einem der Ansprüche 1 bis 4,
**dadurch gekennzeichnet, dass** die Matrize (5) zwei quer zur Richtung der Hauptformänderung verlaufende und beidseitig einer Ausnehmung (7) der Matrize angeordnete Sicken (6) aufweist.

6. Vorrichtung nach einem der Ansprüche 1 bis 5,
**dadurch gekennzeichnet, dass** verschiedene Ziehtiefen fest einstellbar sind.

## Claims

1. Device for determining the quality of a metallic surface of a metallic substrate, comprising a mould, a sheet holder and a die, with which the substrate is formed in order to produce a drawn test surface, wherein the die
(1) is formed so that in the area of the test surface of the formed substrate the main shape change amounts to 7% maximum and the additional shape change amounts to between -2 % and +2 %, preferably close to 0 %, **characterized in that**, the die (1) has a length of at least 400 mm, a width of at least 250 mm and a front face
(2) with a curvature towards the main shape change with a radius of curvature (R₃) from 500 to 2000 mm, preferably 1000 mm.

2. Device according to claim 1, **characterized in that** the edge radius (R₂) between the front face (2) of the die (1) and two side faces (3) of the die, running transversely to the direction of the main shape change, amounts to 20 to 80 mm, preferably 40 mm.

3. Device according to claim 1 or 2, **characterized in that** the edge radius (R₁) between the front face (2) of the die (1) and two side faces (4) of the die, running parallel to the direction of the main shape change, amounts to between 2 and 10 mm, preferably 5 mm.

4. Device according to any one of claims 1 to 3, **characterized in that** the edges between the side faces (3, 4) of the die (1) have a corner radius (R₄) from 50 to 100 mm, preferably 70 mm.

5. Device according to any one of claims 1 to 4, **characterized in that** the mould (5) has two corrugations (6), running transversely to the direction of the main shape change and arranged on both sides of a recess (7) of the mould.

6. Device according to any one of claims 1 to 5, **characterized in that** different drawing depths are permanently adjustable.

## Revendications

1. Dispositif pour la détermination de la qualité d'une surface métallique d'un substrat métallique avec une matrice (5), un support de tôle et un vérin (1) permettant de déformer le substrat, pour réaliser une surface d'essai étirée, dans lequel le vérin (1) est conçu de manière à ce que dans la région de la surface d'essai du substrat déformé, la modification de forme principale représente au maximum 7% et la modification de forme secondaire varie entre -2% et +2%, en se rapprochant de préférence de 0%, **caractérisé en ce que**
le vérin (1) présente une longueur d'au moins 400 mm, une largeur d'au moins 250 mm et une surface frontale (2) avec une courbure dans le sens de la modification de forme principale, avec un rayon de courbure (R₃) de 500 à 2000 mm, de préférence de 1000 mm.

2. Dispositif selon la revendication 1,
**caractérisé en ce que**
le rayon d'arête (R₂) entre la surface frontale (2) du vérin (1) et deux surfaces latérales (3) du vérin s'étendant perpendiculairement au sens de modification de forme principal mesure entre 20 et 80 mm, de préférence 40 mm.

3. Dispositif selon l'une des revendications 1 ou 2, **caractérisé en ce que**
le rayon d'arête (R₁) entre la surface frontale (2) du vérin (1) et deux surfaces latérales (4) du vérin s'étendant parallèlement au sens de modification de forme principal mesure entre 2 et 10 mm, de préférence 5 mm.

4. Dispositif selon l'une des revendications 1 à 3, **caractérisé en ce que**
les bords entre les surfaces latérales (3, 4) du vérin (1) présentent un rayon de coin (R₄) de 50 à 100 mm, de préférence de 70 mm.

5. Dispositif selon l'une des revendications 1 à 4, **caractérisé en ce que**
la matrice (5) comporte deux creux (6) s'étendant perpendiculairement au sens de modification de forme principal et agencés de part et d'autre d'un évidement (7) de la matrice.

6. Dispositif selon l'une des revendications 1 à 5, **caractérisé en ce que**
différentes profondeurs d'étirage peuvent être réglées fixement.
